# EUROPEAN PATENT SPECIFICATION

(11) **EP 0 777 687 B1**
(45) Date of publication and mention of the grant of the patent: **14.06.2006**
(21) Application number: 95929783.9
(22) Date of filing: 25.08.1995
(51) Int. Cl.: C07K 14/575, A61K 38/22

(54) **TREFOIL PEPTIDE DIMER**
DIMERE DES KLEEBLATT-PROTEINS
DIMERES DE PEPTIDES A TROIS BOUCLES

(30) Priority: 26.08.1994 DK 98394
(43) Date of publication of application: 11.06.1997
(73) Proprietor: NOVO NORDISK A/S, 2880 Bagsvaerd (DK)
(72) Inventor: THIM, Lars, 2820 Gentofte (DK); WÖLDIKE, Helle Fabricius, 3540 Lynge (DK); NIELSEN, Per Franklin, 3500 Varlese (DK)
(86) International application number: PCT/DK1995/000343
(87) International publication number: WO 1996/006861

(56) References cited:
- WO-A-92/14837
- FEBS LETTERS, Volume 357, 1995, REBECCA CHINERY et al., "Characterisation of the Single Copy Trefoil Peptides Intestinal Trefoil Factor and pS2 and Their Ability to Form Covalent Dimers", pages 50-54.
- BIOCHEMISTRY, Volume 34, 1995, LARS THIM et al., "Characterization of Human and Rat Intestinal Trefoil Factor Produced in Yeast", pages 4757-4764.
- Thim: (1994) Digestion 55, 353-360.
- Thim: (1989) FEBS Lett. 250, 85-90.
- Thim et al: (1993) FEBS Lett. 318, 345-352.
- Suemori et al: (1991) PNAS, USA 88, 11017-11021.

## Description

### FIELD OF INVENTION

The present invention relates to trefoil peptide dimers, to a method of preparing dimers of trefoil peptides, to pharmaceutical compositions comprising trefoil peptide dimers, and to the use thereof in the treatment of gastrointestinal disorders.

### BACKGROUND OF THE INVENTION

Trefoil peptides form a family of peptides found mainly in association with the gastrointestinal tract. Mammalian trefoil peptides contain one or more characteristic trefoil domains (Thim et al., 1989), each of which is made up of a sequence of 38 or 39 amino acid residues in which 6 half-cystine residues are linked in the configuration 1-5, 2-4 and 3-6, thus forming a characteristic trefoil structure (Thim, 1989).

The mammalian trefoil peptides known at present contain either one or two trefoil domains (for review see Thim, 1994; Poulsom & Wright, 1993; Hoffmann & Hauser, 1993), whereas the frog, Xenopus laevis peptides and proteins containing one (Hauser & Hoffmann, 1991) two (Hauser et al., 1992a) four (Hoffmann, 1988) or six (Hauser & Hoffmann, 1992b) trefoil domains have been described. The mammalian trefoil peptides containing one domain are the breast cancer associated pS2 peptide so far known from human (Jakowlev et al., 1984, Prud'homme et al., 1985) and mouse (Lefebvre et at., 1993) and intestinal trefoil factor so far known from human (Podolsky et al., 1993; Hauser et al., 1993) and rat (Suemori et al., 1991; Chinery et al., 1992). Spasmolytic polypeptide (SP) which contains two trefoil domains has been described from man (Tomasetto et al., 1990), pig (Thim et al., 1982) and mouse (Tomasetto et al., 1990). In humans the three trefoil peptides hpS2, hITF and hSP are all expressed under normal conditions in the gastrointestinal tract: hSP and hps2 in the epithelial mucosal layer of the stomach (Tomasetto et al., 1990; Rio et al., 1988), and hITF in the epithelial mucosal layer of the small intestine and colon (Podolsky et al., 1993). The purification and characterization of hSP produced recombinantly in yeast has also been described (Thin et al., 1993, FEBS Lett. 318(3), pp.345-352).

The physiological function of the trefoil peptides is not very well understood. Increased expression of trefoil peptides in the gastrointestinal tract has been reported in several conditions involving mucosal injury, such as inflammatory bowel disease (Rio et al., 1991; Poulsom et al., 1992; Wright et al., 1993) and ulceration in the stomach and duodenum (Rio et al., 1991; Hanby et al., 1993; Wright et al., 1990). Consequently a mucosal repair function of the trefoil peptides has been suggested (e.g. Wright et al., 1993). Evidence of trefoil peptides promoting mucosal epithelial restitution after injury has recently been provided by Dignass et al., 1994, Playford et al., 1994 and Babyatsky et al., 1994. The mechanism by which trefoil peptides promote their repair function may invole cross-linking of mucin-glyeoproteins to form a viscoelastic gel layer resistant to digestive enzymes (Thim, 1994; Gajhede et al., 1993).

The cloning of rat and human single-domain intestinal trefoil factor and the use of the intestinal trefoil factor in the treatment of gastrointestinal injury are described in WO 92/14837.

### SUMMARY OF THE INVENTION

It has now been found possible to prepare dimers of trefoil factors which have only one trefoil domain and which have interesting pharmacological properties.

Accordingly, the present invention relates to a trefoil peptide containing a single trefoil domain, the peptide being characterized by being in dimer form.

As indicated above, trefoil peptides are believed to contribute to the healing of peptic ulcers and other mucosal injuries by stabilizing the mucus layer of the intestinal tract. The mechanism of this stabilization is at present unknown. However, the X-ray structure of porcine pancreatic spasmolytic polypeptide (PSP) (cf. Gajhede et al., 1993), which has two trefoil domains, shows that the majority of conserved residues contribute to a cleft, 8-10 Å wide, found in each of the trefoil domains. Preliminary docking experiments have shown that the cleft could accommodate part of an oligosaccharide chain, for instance the carbohydrate attached to a mucin glycoprotein. If this is the case, PSP with two such clefts may cross-link mucins, helping them to form a protective gel over the mucosal epithelium. It is not known at present whether trefoil peptides with a single trefoil domain (such as ITF and pS2) form dimers in vivo to exert a similar function, or whether they have a different mechanism of action. However, it is currently believed that dimers of such trefoil peptides may indeed cross-link mucins and therefore be the active form of the peptides.

In another aspect, the present invention relates to a method of preparing a dimer of a trefoil peptide containing a single trefoil domain, the method comprising:culturing a suitable host cell transformed with a DNA sequence encoding a trefoil peptide containing one trefoil domain under conditions permitting production of the peptide; separating the resulting trefoil peptide monomer and trefoil peptide dimer from the culture by a chromatographic procedure ; and recovering the resulting trefoil peptide dimer from the culture.

In a further aspect, the invention relates to a pharmaceutical composition comprising a dimer of a trefoil peptide containing a single trefoil domain, together with a pharmaceutically acceptable diluent or vehicle.

In still further aspects, the invention relates to a dimer of a trefoil peptide containing one trefoil domain for use as a medicament and to the use of a dimer of a trefoil peptide containing one trefoil domain for the preparation of a medicament for the prophylaxis or treatment of gastrointestinal disorders.

### DETAILED DESCRIPTION OF THE INVENTION

The dimer of the trefoil peptide may, in particular, be a dimer of intestinal trefoil factor (ITF) or of breast cancer associated peptide (pS2).

In particular, the trefoil peptide is human ITF,the monomeric amino acid sequence of which is:
ZEYVGLSANQCAVPAKDRVDCGYPHVTPKECNNRGCCFDSRIPGVPWCFKP LQEAECTF
wherein Z is Glu, Gln or pyrGlu,
or
human pS2, the monomeric amino acid sequence of which is :
ZAQTETCTVAPRERQNCGFPGVTPSQCANKGCCFDDTVRGVPWCFYPNTID VPPEEECEF
wherein Z is Glu, Gln or pyrGlu.

In a preferred embodiment, the trefoil peptide dimer of the invention has approximate molecular weight of 13000. In a further preferred embodiment, an ITF dimer of the invention is composed of two ITF monomers linked by a disulphide bond between two crystein residues in position 57 of each monomer. In another preferred embodiment, a pS2 dimer of the invention is composed of two pS2 monomers linked by a disulphide bond between two cysteine residues in position 58 of each monomer. The trefoil peptide dimer is preferably produced by recombinant DNA techniques. To this end, a DNA sequence encoding the trefoil peptide may be isolated by preparing a genomic or cDNA library and screening for DNA sequences coding for all or part of the peptide by hybridization using synthetic oligonucleotide probes in accordance with standard techniques (cf. Sambrook et al., Molecular Cloning: A Laboratory Manual, Cold Spring Harbor Laboratory, Cold Spring Harbor, New York, 1989). For the present purpose, the DNA sequence encoding the peptide is preferably of human origin, i.e. derived from a human genomic DNA or cDNA library.

The DNA sequence encoding the trefoil peptide may also be prepared synthetically by established standard methods, e.g. the phosphoamidite method described by Beaucage and Caruthers, Tetrahedron Letters 22 (1981), 1859 - 1869, or the method described by Matthes et al., EMBO Journal 3 (1984), 801 - 805. According to the phosphoamidite method, oligonucleotides are synthesized, e.g. in an automatic DNA synthesizer, purified, annealed, ligated and cloned in suitable vectors.

The DNA sequence may also be prepared by polymerase chain reaction using specific primers, for instance as described in US 4,683,202, Saiki et al., Science 239 (1988), 487 - 491, or Sambrook et al., *supra.*

The DNA sequence encoding the trefoil peptide is usually inserted into a recombinant vector which may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the trefoil peptide is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the polypeptide.

The promoter may be any DNA sequence which shows transcriptional activity in the host cell of choice and may be derived from genes encoding proteins either homologous or heterologous to the host cell.

Examples of suitable promoters for directing the transcription of the DNA encoding the trefoil peptide in mammalian cells are the SV40 promoter (Subramani et al., Mol. Cell Biol. 1 (1981), 854 -864), the MT-1 (metallothionein gene) promoter (Palmiter et al., Science 222 (1983), 809 - 814) or the adenovirus 2 major late promoter.

An example of a suitable promoter for use in insect cells is the polyhedrin promoter (US 4,745,051; Vasuvedan et al., FEBS Lett. 311, (1992) 7 - 11), the P10 promoter (J.M. Vlak et al., J. Gen. Virology 69, 1988, pp. 765-776), the *Autographa californica* polyhedrosis virus basic protein promoter (EP 397 485), the baculovirus immediate early gene 1 promoter (US 5,155,037; US 5,162,222), or the baculovirus 39K delayed-early gene promoter (US 5,155,037; US 5,162,222).

Examples of suitable promoters for use in yeast host cells include promoters from yeast glycolytic genes (Hitzeman et al., J. Biol. Chem. 255 (1980), 12073 - 12080; Alber and Kawasaki, J. Mol. Appl. Gen. 1 (1982), 419 - 434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., Nature 304 (1983), 652 - 654) promoters.

Examples of suitable promoters for use in filamentous fungus host cells are, for instance, the ADH3 promoter (McKnight et al., The EMBO J. 4 (1985), 2093 - 2099) or the tpiA promoter. Examples of other useful promoters are those derived from the gene encoding *A. oryzae* TAKA amylase, *Rhizomucor miehei* aspartic proteinase, A. *niger* neutral α-amylase, *A. niger* acid stable α-amylase, *A. niger* or *A. awamori* glucoamylase (gluA), *Rhizomucor miehei* lipase, *A. oryzrre* alkaline protease, *A. oryzae* triose phosphate isomerase or *A. nidulans* acetamidase. Preferred are the TAKA-amylase and gluA promoters. Suitable promoters are mentioned in, e.g. EP 238 023 and EP 383 779.

The DNA sequence encoding the trefoil peptide may also, if necessary, be operably connected to a suitable terminator, such as the human growth hormone terminator (Palmiter et al., Science 222, 1983, pp. 809-814) or (for fungal hosts) the TPI1 (Alber and Kawasaki, J. Mol. Appl. Gen. 1. 1982, pp. 419-434) or ADH3 (McKnight et al., The EMBO J. 4, 1985, pp. 2093-2099) terminators. The vector may further comprise elements such as polyadenylation signals (e.g. from SV40 or the adenovirus 5 Elb region), transcriptional enhancer sequences (e.g. the SV40 enhancer) and translational enhancer sequences (e.g. the ones encoding adenovirus VA RNAs).

The recombinant vector may further comprise a DNA sequence enabling the vector to replicate in the host cell in question. An example of such a sequence (when the host cell is a mammalian cell) is the SV40 origin of replication.

When the host cell is a yeast cell, suitable sequences enabling the vector to replicate are the yeast plasmid 2µ replication genes REP 1-3 and origin of replication.

The vector may also comprise a selectable marker, e.g. a gene the product of which complements a defect in the host cell, such as the gene coding for dihydrofolate reductase (DHFR) or the *Schizosaccharomyces pombe* TPI gene (described by P.R. Russell, Gene 40, 1985, pp. 125-130), or one which confers resistance to a drug, e.g. ampicillin, kanamycin, tetracyclin, chloramphenicol, neomycin, hygromycin or methotrexate. For filamentous fungi, selectable markers include amdS, pyrG, argB, niaD or sC.

To direct a trefoil peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the trefoil peptide in the correct reading frame. Secretory signal sequences are commonly positioned 5' to the DNA sequence encoding the peptide. The secretory signal sequence may be that normally associated with the peptide or may be from a gene encoding another secreted protein.

For secretion from yeast cells, the secretory signal sequence may encode any signal peptide which ensures efficient direction of the expressed trefoil peptide into the secretory pathway of the cell. The signal peptide may be naturally occurring signal peptide, or a functional part thereof, or it may be a synthetic peptide. Suitable signal peptides have been found to be the α-factor signal peptide (cf. US 4,870,008), the signal peptide of mouse salivary amylase (cf. O. Hagenbuchle et al., Nature 289, 1981, pp. 643-646), a modified carboxypeptidase signal peptide (cf. L.A. Valls et al., Cell 48, 1987, pp. 887-897), the yeast BAR1 signal peptide (cf. WO 87/02670), or the yeast aspartic protease 3 (YAP3) signal peptide (cf. M. Egel-Mitani et al., Yeast 6, 1990, pp. 127-137).

For efficient secretion in yeast, a sequence encoding a leader peptide may also be inserted downstream of the signal sequence and uptream of the DNA sequence encoding the trefoil peptide. The function of the leader peptide is to allow the expressed peptide to be directed from the endoplasmic reticulum to the Golgi apparatus and further to a secretory vesicle for secretion into the culture medium (i.e. exportation of the trefoil peptide across the cell wall or at least through the cellular membrane into the periplasmic space of the yeast cell). The leader peptide may be the yeast α-factor leader (the use of which is described in e.g. US 4,546,082, US 4,870,008, EP 16 201, EP 123 294, EP 123 544 and EP 163 529). Alternatively, the leader peptide may be a synthetic leader peptide, which is to say a leader peptide not found in nature. Synthetic leader peptides may, for instance, be constructed as described in WO 89/02463 or WO 92/11378.

For use in filamentous fungi, the signal peptide may conveniently be derived from a gene encoding an *Aspergillus* sp. amylase or glucoamylase, a gene encoding a *Rhizomucor miehei* lipase or protease or a *Humicola lanuginosa* lipase. The signal peptide is preferably derived from a gene encoding *A. oryzae* TAKA amylase, *A. niger* neutral α-amylase, *A*. *niger* acid-stable amylase, or *A*. *niger* glucoamylase. Suitable signal peptides are disclosed in, e.g. EP 238 023 and EP 215 594.

For use in insect cells, the signal peptide may conveniently be derived from an insect gene (cf. WO 90/05783), such as the lepidopteran *Manduca sexta* adipokinetic hormone precursor signal peptide (cf. US 5,023,328).

The procedures used to ligate the DNA sequences coding for the trefoil peptide, the promoter and optionally the terminator and/or secretory signal sequence, respectively, and to insert them into suitable vectors containing the information necessary for replication, are well known to persons skilled in the art (cf., for instance, Sambrook et al., Molecular Cloning: A Laboratory Manual, Cold Spring Harbor, New York, 1989).

The host cell into which the DNA sequence encoding the trefoil peptide is introduced may be any cell which is capable of producing the peptide in dimer form and includes yeast, fungi and higher eukaryotic cells.

Examples of suitable mammalian cell lines are the COS (ATCC CRL 1650), BHK (ATCC CRL 1632, ATCC CCL 10), CHL (ATCC CCL39) or CHO (ATCC CCL 61) cell lines. Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet, 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841 - 845.

Examples of suitable yeasts cells include cells of *Saccharomyces* spp. or *Schizosaccharomyces* spp., in particular strains of *Saccharomyces cerevisiae* or *Saccharomyces kluyveri.* Methods for transforming yeast cells with heterologous DNA and producing heterologous polypeptides therefrom are described, e.g. in US 4,599,311, US 4,931,373, US 4,870,008, 5,037,743, and US 4,845,075. Transformed cells are selected by a phenotype determined by a selectable marker, commonly drug resistance or the ability to grow in the absence of a particular nutrient, e.g. leucine. A preferred vector for use in yeast is the POT1 vector disclosed in US 4,931,373. The DNA sequence encoding the trefoil peptide may be preceded by a signal sequence and optionally a leader sequence , e.g. as described above. Further examples of suitable yeast cells are strains of *Kluyveromyces,* such as *K.* lacris, *Hansenula, e.g. H. polymorpha,* or *Pichia,* e.g. *P. pastoris* (cf. Gleeson et al., J. Gen. Microbiol. 132, 1986, pp. 3459-3465; US 4,882,279).

Examples of other fungal cells are cells of blamencous fungi, e.g. *Aspergillus* spp., *Neurospora* spp., *Fusarium* spp. or *Trichoderma* spp., in particular strains of *A. oryzae, A. nidulans* or *A. niger.* The use of *Aspergillus* spp. for the expression of proteins is described in, e.g., EP 272 277, EP 238 023, EP 184 438 The transformation of F. *oxysporum* may, for instance, be carried out as described by Malardier et al., 1989, Gene 78: 147-156. The transformation of *Trichoderma* spp. may be performed for instance as described in EP 244 234.

When a filamentous fungus is used as the host cell, it may be transformed with the DNA construct of the invention, conveniently by integrating the DNA construct in the host chromosome to obtain a recombinant host cell. This integration is generally considered to be an advantage as the DNA sequence is more likely to be stably maintained in the cell. Integration of the DNA constructs into the host chromosome may be performed according to conventional methods, e.g. by homologous or heterologous recombination.

Transformation of insect cells and production of heterologous polypeptides therein may be performed as described in US 4,745,051; US 4,879,236; US 5,155,037; 5,162,222; EP 397,485.The insect cell line used as the host may suitably be a *Lepidoptera* cell line, such as *Spodoptera frigiperda* cells or *Trichoplusia ni* cells (cf. US 5,077,214). Culture conditions may suitably be as described in, for instance, WO 89/01029 or WO 89/01028, or any of the aforementioned references.

The transformed or transfected host cell described above is then cultured in a suitable nutrient medium under conditions permitting the expression of the trefoil peptide, after which all or part of the resulting peptide may be recovered from the culture in dimer form. The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The trefoil peptide produced by the cells may then be recovered from the culture medium by conventional procedures, including separating the host cells from the medium by centrifugation or filtration, precipitating the proteinaceous, components of the supernatant or filtrate by means of a salt, e.g. ammonium sulphate, purification by a variety of chromatographic procedures, e.g. ion exchange chromatography, gelfiltration chromatography, affinity chromatography, or the like, dependent on the type of polypeptide in question. In a method of the invention for preparing a trefoil peptide dimer according to the invention, the trefoil peptide monomer and trefoil peptide dimer from the culture are separated by a chromatographic procedure, whereafter the dimer is recovered

In the pharmaceutical composition of the invention, the trefoil peptide dimer may be formulated by any of the established methods of formulating pharmaceutical compositions, e.g. as described in Remington's Pharmaceutical Sciences. 1985. The composition may be in a form suited for systemic injection or infusion and may, as such, be formulated with sterile water or an isotonic saline or glucose solution. The compositions may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc.

The pharmaceutical composition of the present invention may also be nasal, transdermal or rectal administration. The pharmaceutically acceptable vehicle or diluent employed in the composition may be any conventional solid carrier. Examples of solid carriers are lactose, terra alba, sucrose, talc, gelatin, agar, pectin, acacia, magnesium stearate and stearic acid. Similarly, the vehicle or diluent may include any sustained release material known in the art, such as glyceryl monostearate or glyceryl distearate, alone or mixed with a wax. The amount of a solid carrier will vary widely, but will usually be from about 25 mg to about 1 g.

The concentration of the trefoil peptide dimer in the composition may vary widely, i.e. from from about 5% to about 100% by weight. A preferred concentration is in the range of 50-100% by weight. A unit dosage of the composition may typically contain from about 1 mg to about 200 mg, preferably from about 25 mg to about 75 mg, in particular about 50 mg, of the dimer.

As indicated above, the trefoil peptide dimer of the invention is believed to be the active form of the peptide. As such it is contemplated to be advantageous to use for prevention or treatment of gastrointestinal disorders. More specifically, it is contemplated for use in the treatment of gastric or peptic ulcers, inflammatory bowel disease, Crohn's disease or injury to the intestinal tract caused by radiation therapy, bacterial or other infections. The dosage of the polypeptide administered to a patient will vary with the type and severity of the condition to be treated, but is generally in the range of 0.1-1.0 mg/kg body weight. A further aspect of the invention relates to the use of a trefoil peptide dimer of the invention for the preparation of a medicament for the prophylaxis or treatment of gastro intestinal disorders, such as one of the latter mentioned disorders.

### BRIEF DESCRIPTION OF THE DRAWINGS

The present invention is described in further detail in the example with reference to the appended drawings, which are as follows :
- Fig. 1: Proposed structure of human intestinal trefoil factor ITF. The primary amino acid sequence is taken from Hauser et al., 1993, and the disulphide bonds are placed in homology to PSP and pS2 (Thim, 1988).
- Fig. 2: Reversed phase HPLC on a Vydac 214TP54 column of supernatant from yeast strain HW756 expressing rat ITF.
- Fig. 3: Ion exchange chromatography on a Fast Flow SP-Sepharose column of partially purified human ITF. The amount of hITF (monomer) and hITF (dimer) were determined by analytical HPLC. The bars indicate the fractions pooled for further purification of the monomer and dimer forms. The dashed line shows the concentration of NaCl in the eluting solvent.
- Fig. 4: Reversed-phase HPLC on a Vydac 214TP54 C4 column of purified rat ITF, dimer (A), human ITF (monomer) B, and human ITF (dimer C). The dashed lines show the concentration of acetonitrile in the eluting solvent.
- Fig. 5: Reconstructed mass spectra of purified rat ITF (dimer) (A), human ITF (monomer) (B) and human ITF (dimer)(C).

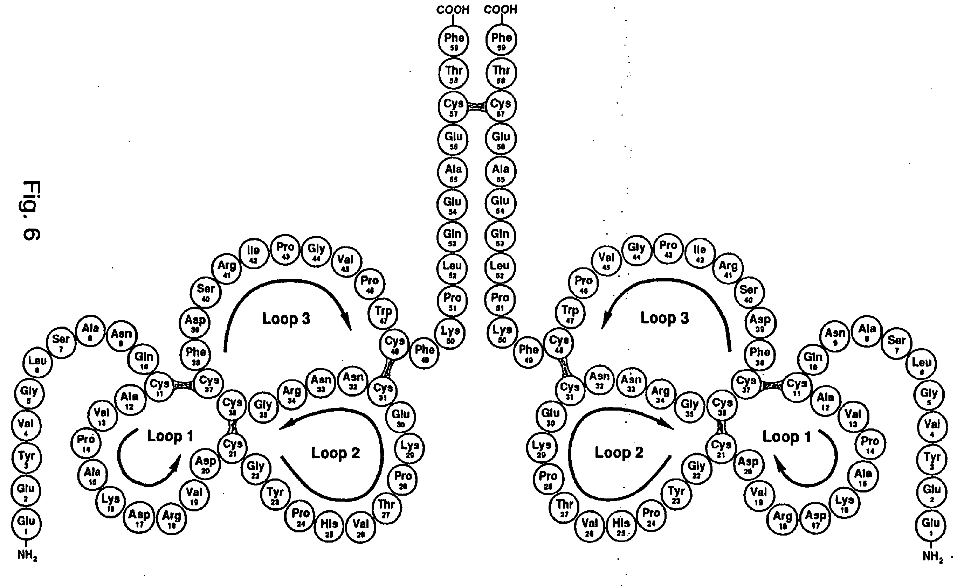

- Fig. 6: Structure of the dimer form of human ITF.
- Fig. 7: Restriction map pf plasmid KFN 1003.
- Fig. 8: Construction of plasmid pHW756.
- Fig. 9: Construction of plasmid pHW1066.

### EXAMPLE

### MATERIALS AND METHODS

### Cloning of rat ITF (rITF) and human ITF (hITF)

The cloning of rat and human ITF was carried out as described in WO 92/14837 and as described by Suemori et al., 1991, and Chinery et al., 1992 (rat ITF), and Podolsky et al., 1993, and Hauser et al., 1993 (human ITF).

### Construction of rITF and hITF expression plasmids

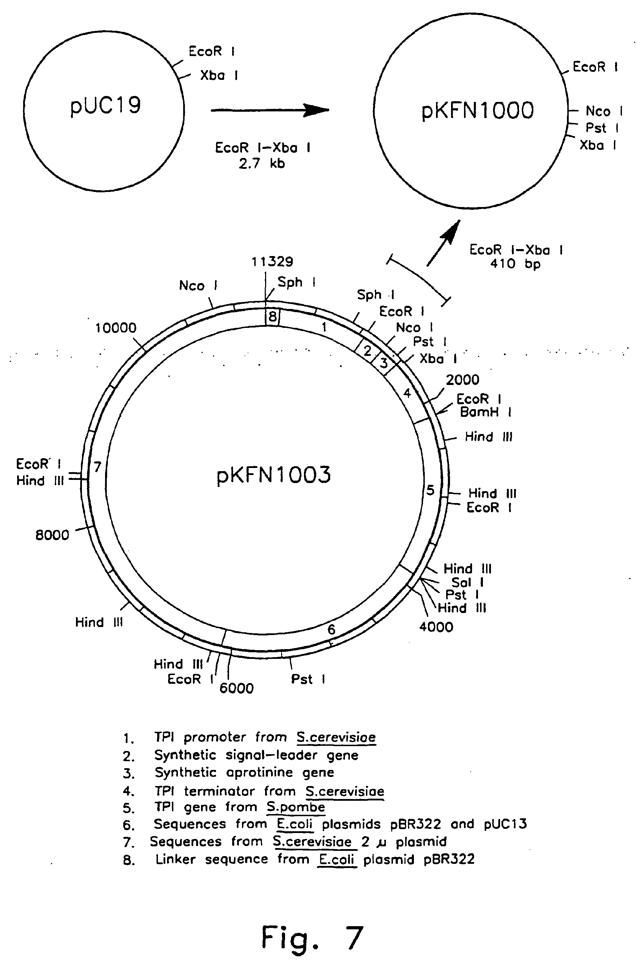

The expression plasmids pHW756 for secretion of rat ITF and pHW1066 for secretion of human ITF were constructed as outlined in Fig. 7-9. The yeast expression vector pKFN1003 (described in WO 90/10075) is a derivative of plasmid CPOT (Kawasaki, G. International Conference on Yeast Genetics and Molecular Biology, Sept.17-24, 1984, Edinburgh, Scotland, Abstr. P15.). It has Schizosaccharomyces pombe TPI gene (POT) as selection marker (Russell, P.R., Gene 40 (1985), 125-130), and the S.cerevisiae triose phosphate isomerase (TPI) promoter and terminator for control of expression (Alber, T. and Kawasaki, G. J.Mol.Appl.Genet. 1 (1982), 419-434).

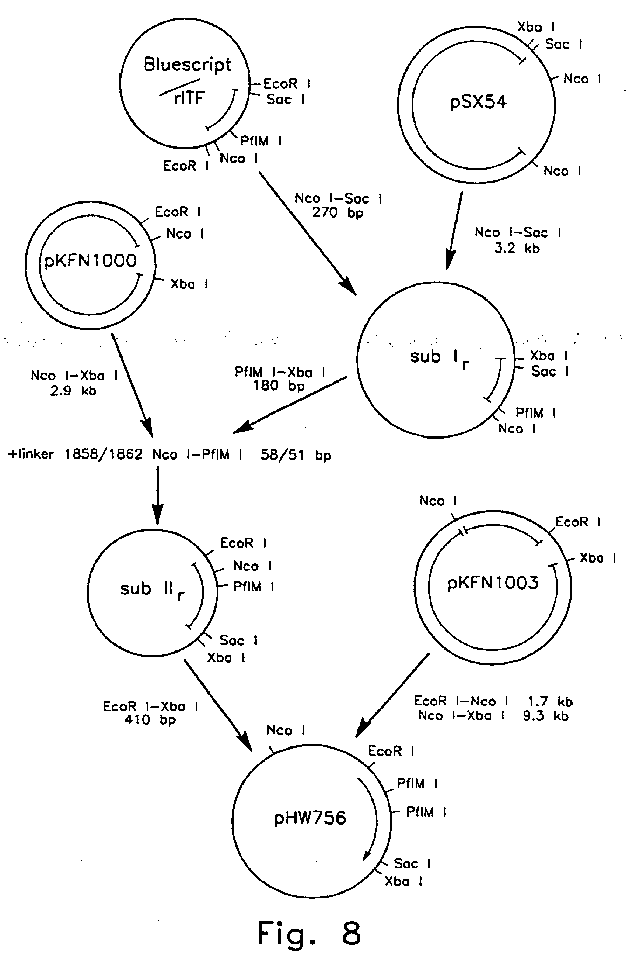

The rat ITF gene was initially cloned into Blilescript. II KS(-) (Stratagene), from which it was propagated according to Fig. 8. The helper vector pSX54 providing useful cloning sites, is composed of pUC18 and pDN1050 (Diderichsen, B., Poulsen, G.B., Jørgensen, S.T. Plasmid 30 (1993), 312-315). The synthetic DNA linker Ncol-Pf1M1 has the following sequence :
- 1858:: 5'-CATGGCTGAAAGATTGGAAAAGAGACAAGAGTTCGTTGGTTTGTCTCCATCCCAATGT-3' 58 bp
- 1862:: 5'-TTGGGATGGAGACAAACCAACGAACTCTTGTCTCTTTTCCAATCTTTCAGC-3' 51 bp

The linker codes for the C-terminal 8 amino acids of the leader sequence as described in Thim, L., Norris, K., Norris, F., Nielsen, P.F., Bjørn. S., Christensen, M., Petersen, J. FEBS Lett. 318, (1993), 345-352, with a few changes in codon choice, and the N-terminal part of the rat ITF gene : QEFVGLSPSQC. The amino acid sequence of the signal and leader is as described ibid. :
MKAVFLVLSLIGFCWAQPVTGDESSVEIPEESLIIAENTTLANVAMAERLEKR.

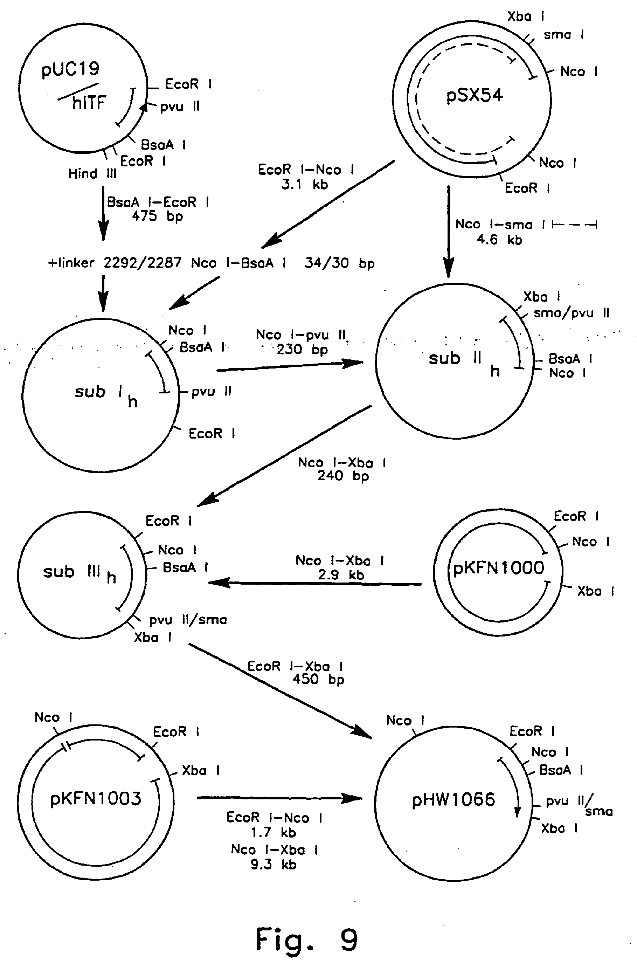

The human ITF gene was cloned into PUC19 and propagated as described in Fig. 9. The synthetic DNA linker Ncol-BsaAl has the following sequence:
2292 : 5'-CATGGCTGAAAGATTGGAAAAGAGAGAAGAATAC-3' 34 bp
2287: 5'-GTATTCTTCTCTCTTTTCCAATCTTTCAGC-3' 30 bp

The linker codes for the C-terminal 8 amino acids of the leader as described for the rITF con-struction and for the N-terminal 3 amino acids of the hITF gene : EEY. The signal and leader are the same as above.

The expression plasmids were transformed into S.cerevisiae strain MT 663 (E2-7B X E11-36 a/α, △tpi/△tpi, pep 4-3/pep 4-3) by selection for growth on glucose as the sole carbon source.

The yeast transformants expressing rat ITF and human ITF were named HW756 and HW1066, respectively.

### Fermentations

The transformants described above were cultivated at 30°C for 72h in yeast peptone dextrose (YPD) medium (Sherman et al., 1981) supplied with additional yeast extract (60 Gram/L). OD values at 660 nm of 153 and 232 for HW756 (rITF) and HW1066 (hITF), respectively, were reached at the end of the fermentations. The pH was adjusted to 2.5 with 1M phosphoric acid at the end of the fermentation, and the yeast cells were removed by centrifugation at 3000 rpm for 15 min.

### Purification of recombinant rITF

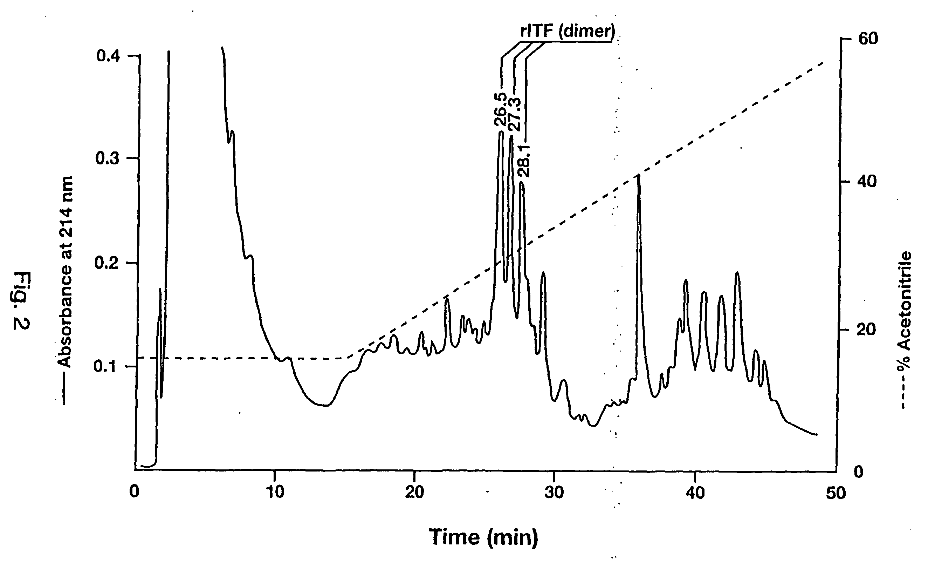

The concentration of rITF in the yeast fermentation broth and fractions obtained during the purification was measured by analytical HPLC. Aliquots (usually 50-200 *µ*L) were injected onto a Vydac 214TP54 reverse-phase C4 HPLC column (0.46 x 25 cM) equilibrated at 30°C at a flow rate of 1.5 mL/min. with 0.1% (v/v) TFA in 15% (v/v) acetonitrile. After 10 min of isocratic elution, the concentration of acetonitrile in the eluting solvent was raised to 55% over 40 min. Absorbance was measured at 214 nm. Three peaks eluting at 26.5 min., 27.3 min. and 28.2 min. (Fig. 2) was found to represent dimer forms of rITF. The peptides were quantified using a calibrated hSP standard (Thim et al., 1993).
The expression level for recombinant rat ITF in the present yeast system was 113 mg/L.

From a 10 L fermenter, 8.7 L of fermentation broth was isolated by centrifugation. The supernatant was diluted with 14.8 L of distilled water to lower the conductivity. The sample was pumped onto a Fast Flow S-Sepharose (Pharmacia) column (5 x 42 cM) with a flow rate of 600 mL/h. Prior to the application, the column was equilibrated in 50 mM formic acid buffer, pH 3.7. Rat ITF was eluted from the column by 50 mM formic acid, pH 3.7 containing 50 mM NaCl. Fractions of 100 mL were collected at a flow rate of 600 mL/h and analysed for the content of rITF. Fractions from the previous step containing rITF were pooled (2.3 L) and pumped onto an Amberchrome (G-71) column (5 x 10 cM). Prior to the application, the column was equilibrated in 10 mM ammonium acetate buffer, pH 4.8, at a flow rate of 0.5 L/h. After application, the column was washed with 0.5 L of equilibration buffer and eluted with 10 mM ammonium acetate buffer pH 4.8 containing 60% (v/v) of ethanol at a flow rate of 0.1 L/h. Fractions of 10 mL were collected and pooled according to their content of rITF. The ethanol concentration in the pool was increased from 60% (v/v) to 87% (v/v) by the addition of 2 volumes of ethanol (99.9%, v/v) and rITF was precipitated by cooling to resulting mixture to minus 25°C for 16h.

The precipitate was collected by centrifugation for 1 h at 10,000 g at -25°C and redissolved at room temperature in 130 mL of 20 mM formic acid pH 3.0. The sample was pumped onto a Fast Flow SP-Sepharose (Pharmacia) column (5 x 20 cM) with a flow rate of 50 mL/h. Prior to the application, the column was equilibrated with 20 mM formic acid, pH 3.0. Peptides were eluted from the column by a linear gradient between 1.5 L of 50 mM formic acid, pH 3.0 and 1.5 L of formic acid, pH 3.0 containing 0.5 M NaCl. Fractions (10 mL) were collected at a flow rate of 80 mL/h and the absorbance was measured at 280 nm. Fractions were assayed for the content of rITF. Fractions corresponding to rITF were pooled. Rat ITF was further purified by preparative HPLC. Pooled fractions (900 mL) were pumped onto a Vydac 214TP1022 C4 preparative HPLC column (2.2 x 25 cM) equilibrated in 0.1% (v/v) TFA. The peptides were eluted at 25°C and at a flow rate of 5 mL/min. with a linear gradient (540 mL) formed from MeCN/H₂O/TFA (10:89.9:0.1, v/v/v) and MeCN/H₂O/TFA (65:34.9:0.1, v/v/v). UV absorption was monitored at 280 nm and fractions corresponding to 10 mL were collected and analysed for the content of rITF. Fraction containing rITF were pooled and the volume reduced to 30% by vacuum centrifugation. From the resulting pool, rITF was isolated by lyophilization. The total yield of rITF from 8.7 L of fermentation medium was 236 mg corresponding to an overall purification yield of 24%.

### Purification of recombinant hITF

The concentration of hITF in the yeast fermentation broth and fraction, obtained during the purification, was measured in an HPLC system identical to the one described for rITF. In this system, two peaks eluting at 21.2 min. and 27.1 min. were found by mass spectrometry and sequence analysis to represent a dimer and a monomer form of hITF. The expression level for recombinant human ITF in the present yeast system was 90 mg/l.

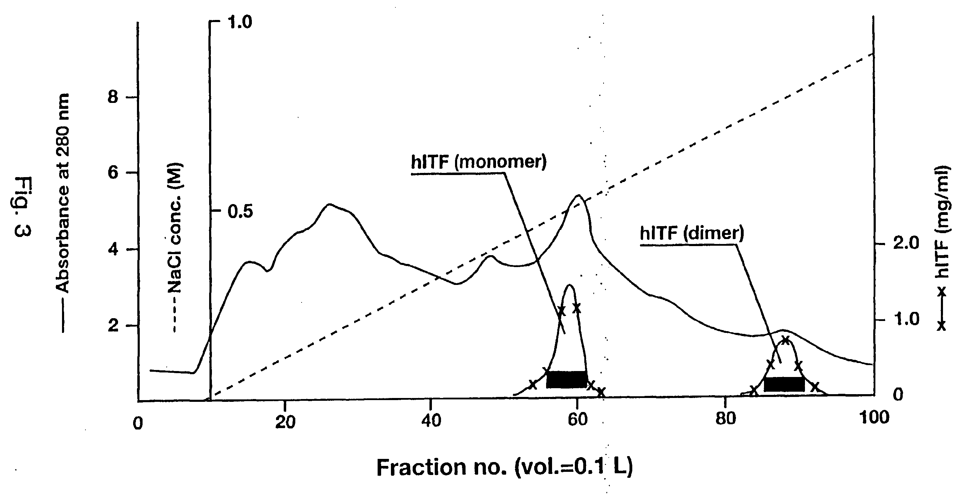

From a 10 L fermenter, 8.0 L of fermentation broth were isolated by centrifugation. The sample was dialysed 3 times (each time in 24 h) against 40 L of 10 mM formic acid, pH 2.5. The sample was pumped (0.25 L/h) onto an SP-Sepharose Fast Flow (Pharmacia) column (5 x 40 cM). The column was washed with 5 L of 20 mM formic acid, pH 2.5, and eluted with a linear gradient formed by 5 L of 20 mM formic acid, pH 2.5 and 5 L of formic acid, pH 2.5 containing 1 M of NaCl. Fractions of 100 mL were collected and analysed for the content of hITF (Fig. 3). Two forms of hITF were eluted from the column: one representing a monomer form of hITF (eluting at 0.5 M of NaCI) and one representing a dimer form of hITF (eluting at 0.78 M of NaCl). Fraction corresponding to the two forms were pooled separately.

Each fraction was divided into three equal parts (volume: approx. 700 mL) and pumped onto a Vydac 214TP1022 C4 column (2.2 x 25 cM) equilibrated in 0.1% (v/v) TFA. The peptides were eluted at a flow rate of 4 mL/min. with a linear gradient (540 mL) between MeCN/H₂O/TFA (10:89.9:0.1, v/v/v) and MeCN/H₂O/TFA (65:34.9:0.1, v/v/v). UV absorption was monitored at 280 nm and fractions corresponding to 10 mL were collected and analysed for the content of hITF.

Fraction from the previous step containing hITF (monomer) and hITF (dimer) were pooled separately and the pH was adjusted to 3.0. The samples were applied separately onto an SP-Sepharose HiLoad 16/10 (Pharmacia) column (1.6 x 10 cM) equilibrated in 20 mM formic acid, pH 3.0 containing 40% (v/v) ethanol. The column was washed with 80 mL of equilibration buffer and eluted at a flow rate of 4 mL/min. with a linear gradient between 200 mL of 20 mM formic acid, pH 3.0, 40% (v/v) ethanol and 200 ml of 20 mM formic acid, pH 3.0, 40% (v/v) containing 1 M NaCl. Fractions (5 mL) were collected and analysed for the content of hITF.

Fractions containing hITF (monomer) and hITF (dimer), respectively, were pooled and the peptide content precipitated by adjusting the ethanol concentration to 90% (v/v) and cooling the mixture for 72h at -25°C. The precipitate was collected by centrifugation and lyophilized. The total yield from 8L of fermentation broth was 256 mg hITF (monomer) and 133 mg hITF (dimer) corresponding to an overall purification yield of 50% and 65%, respectively, for the monomer and dimer forms.

### 5 Characterization of recombinant rITF and hITF

After hydrolysis in 6 M HCl at 110°C in vacuum-sealed tubes for 24, 48 and 96 h, the samples (50 *µ*g) were analysed on a Beckman (Model 121 MB) automatic amino acid analyser. Half-cystine was determined as the S-β-(4-pyridylethyl) derivative, after reduction of the disulphide bonds by tributylphosphine (Rüegg & Rudinger, 1974), followed by coupling with 4-vinylpyridine (Friedman et al., 1970). Hydrolyses of 4-vinylpyridine-treated samples were performed by 4 M methanesulphonic acid or 3 M mercaptoethanesulphonic acid at 110°C for 24 h as described above. Amino acid sequence analysis was determined by automated Edman degradation using an Applied Biosystems Model 470A gas-phase sequencer (Thim et al., 1987).

Mass spectrometry analysis was performed using an API III LC/MS/MS system (Sciex, Thornhill, Ont., Canada). The triple quadrupole instrument has a mass-to-charge (m/z) range of 2400 and is fitted with a pneumatically assisted electrospray (also referred to as ion-spray) interface (Bruins et al.; 1987; Covey et al., 1988). Sample introduction was done by a syringe infusion pump (Sage Instruments, Cambridge, MA) through a fused capillary (75 *µ*m i.d.) with a liquid flow rate set at 0.5-1 *µ*l/min*.* The instrument m/z scale was calibrated with the singly-charged ammonium adduct ions of poly(propylene glycols) (PPGs) under unit resolution. The accuracy of mass measurements is generally better than 0.02%.

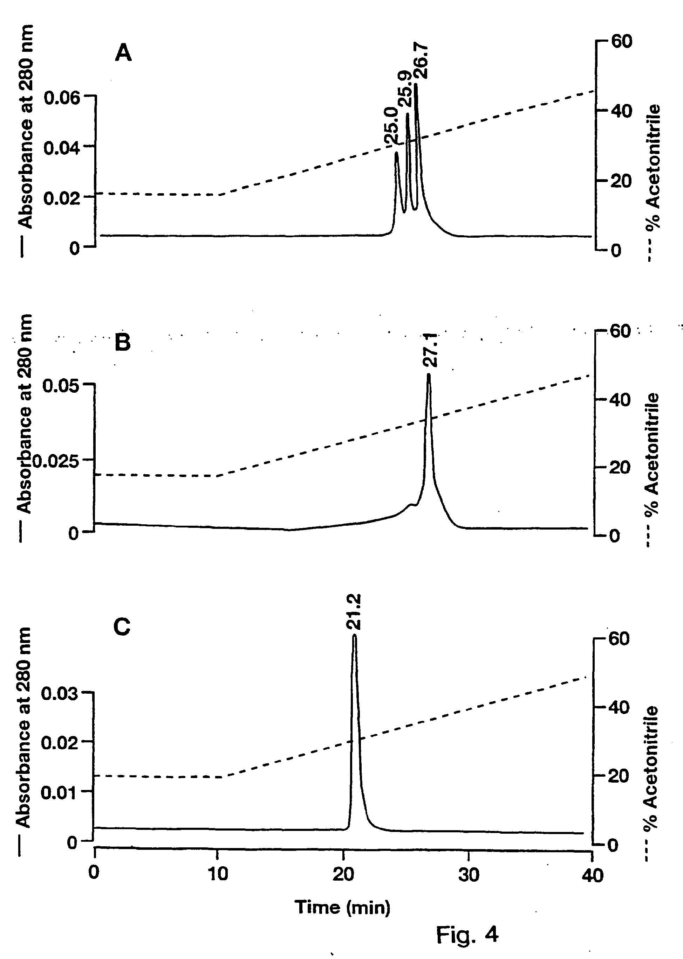

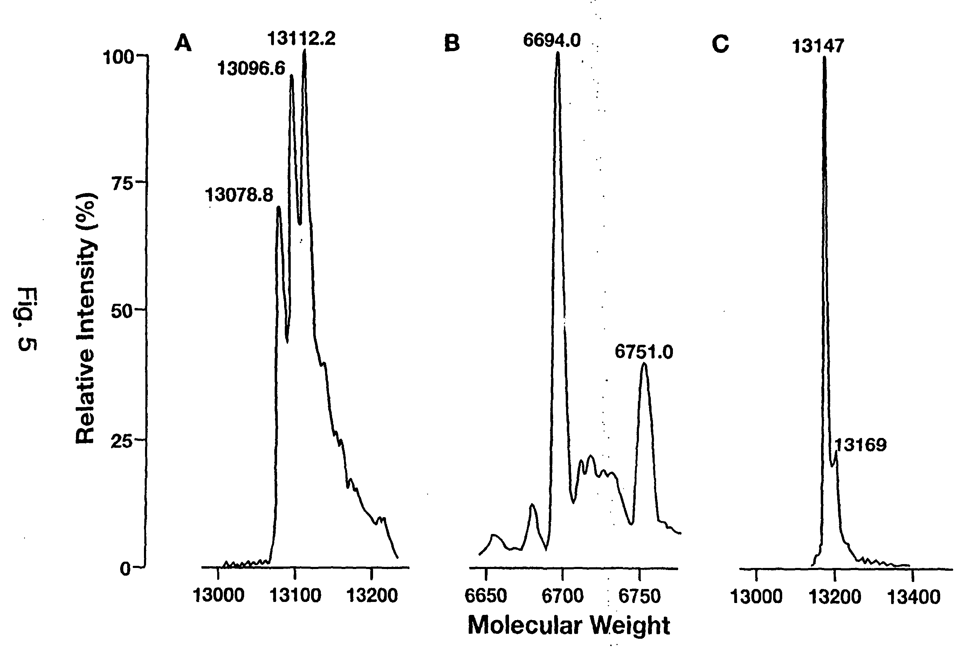

Fig. 4 shows the analytical HPLC chromatograms obtained on the purified rITF (Fig. 4A) and hITF (Fig. 4B and 4C). Recombinant rITF contains a mixture of 3 closely related peptides, and no attempts were made to separate these forms. When analysed by electrospray mass spectrometry, three dominating molecular weights were found, corresponding to: 13112.2, 13096.6 and 13078.8 (Fig. 5A). The calculated molecular weight of rat ITF in a monomer form in which Cys-57 contains a free -SH group is 6558.3. The calculated molecular weight of rat ITF in a dimer form (e.g. an S-S bridge is established between two Cys-57) is 13114.6. From the molecular weights found for the recombinant rat ITF it is clear that all three peptides represent dimer forms of rITF. From other trefoil peptides in which the N-terminal amino acid residue is GIn, e.g. PSP (Thim et al., 1985 and Tomasetto et al., 1990) it is known that this residue has a tendency to cyclize to form a pyrrolidone carboxylic acid (pyrGlu). In the case of rat ITF having a predicted N-terminal sequence of Gln-Glu-Phe-Val-Gly, it seems reasonable to assume that the N-terminal Gln could also cyclize to form pyrGlu. Such a derivatization would result in a decrease in the molecular weight of rat ITF (dimer) on 17 (one pyrGlu) or 34 (two pyrGlu) mass unit, respectively. The observed molecular weights on 13096.6 and 13078.8 (Fig. 5A) correspond to the dimer form of rat ITF in which one, respectively two, N-terminal Gln residues have cyclized. The calculated molecular weight of these forms are 13097.6 and 13080.6 being in good agreement with the experimentally determined values. Thus from the HPLC (Fig. 4A) and mass analysis (Fig. 5A) it is concluded that the recombinant rat ITF consists of 3 different dimer forms: one containing 2 N-terminal Gln, one containing 1 N-terminal Gln and 1 N-terminal pyrGlu and one containing 2 N-terminal pyrGlu. Table I shows the amino acid composition of rat ITF being in good agreement with the expected values.

Figs. 5A and 5B show the purity of hITF (monomer) and hITF (dimer), respectively as analysed by analytical HPLC. The dimer form (Fig. 5C) looks relatively pure whereas the monomer form (Fig. 5B) seems to be contaminated with material eluting in front of peptide. However, upon rechromatography of material eluting in the main peak, a similar chromatogram was obtained (results not shown). This seems to indicate an atypical behaviour of the hITF (monomer) on reverse phase columns rather than impurities. We have previously observed a similar behaviour of highly purified porcine PSP as well as highly purified recombinant hSP (Thim et al., 1993).

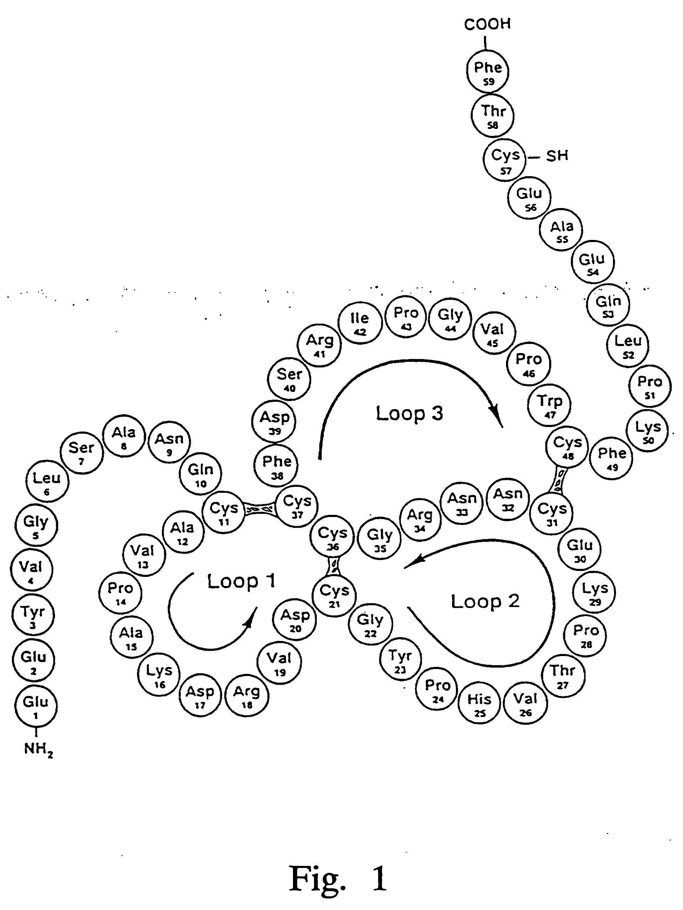

Mass spectrometry analysis of the hITF (monomer) shows a molecular weight of the dominating peak corresponding to 6694.0 (Fig. 5B). The molecular weight, as calculated from the amino acid sequence (Fig. 1), is 6574.4 assuming that Cys-57 exists on -SH form. The amino acid sequence analysis (Table II) shows the expected N-terminal sequence of Glu-Glu-Tyr-Val-Gly-. The amino acid composition analysis (Table I) shows the expected values except for the presence of 7.3 (8) cysteines. An additional cysteine linked to Cys-57 of hITF monomer would increase the molecular weight to 6694.7 which is very close to the value determined by mass spectrometry (6694.0). It is, therefore, assumed that in the hITF (monomer), Cys-57 is disulphide-linked to an additional cysteine. The minor molecular weight peak in the mass spectrum (Fig. 5B) may represent another derivative of Cys-57 or may be an impurity in the preparation.

The calculated molecular weight of hITF (dimer), in which two monomers are linked by a disulphide bond between two Cys-57 residues, is 13146.8. This is in good agreement with the value determined by mass spectrometry (13147, Fig. 5C). The other peak in the mass spectrum (13169) probably represents the Na⁺ adduct of hITF (dimer). The sequence analysis (Table I) as well as the amino acid composition analysis (Table II) are also in good agreement with the expected values.

**Table I Amino acid composition of rat ITF (dimer), human ITF (monomer) and human ITF (dimer)**

| Amino acid | Rat ITF (dimer) | | Human ITF (monomer) | | Human ITF (dimer) | |
|---|---|---|---|---|---|---|
| Asx | 11.92 | (12) | 6.00 | (6) | 12.01 | (12) |
| Thr^{a} | 8.00 | (8) | 2.03 | (2) | 4.01 | (4) |
| Ser^{a} | 9.86 | (10) | 2.04 | (2) | 4.01 | (4) |
| Glx | 15.98 | (16) | 6.92 | (7) | 14.00 | (14) |
| Pro^{b} | 12.48 | (12) | n.d.^{c} | (6) | n.d.^{c} | (12) |
| Gly | 6.02 | (6) | 4.20 | (4) | 8.09 | (8) |
| Ala | 2.04 | (2) | 3.91 | (4) | 7.90 | (8) |
| Val | 11.92 | (12) | 4.92 | (5) | 9.86 | (10) |
| Met | 1.80 | (2) | 0.00 | (0) | 0.00 | (0) |
| Ile | 1.98 | (2) | 1.17 | (1) | 2.13 | (2) |
| Leu | 3.92 | (4) | 2.03 | (2) | 3.99 | (4) |
| Tyr^{b} | 2.02 | (2) | 1.94 | (2) | 3.92 | (4) |
| Phe | 7.88 | (8) | 2.93 | (3) | 5.94 | (6) |
| Lys | 2.14 | (2) | 3.07 | (3) | 6.09 | (6) |
| His | 0.00 | (0) | 0.99 | (1) | 2.03 | (2) |
| Trp | 2.16 | (2) | n.d.^{c} | (1) | n.d.^{c} | (2) |
| Arg | 3.94 | (4) | 2.96 | (3) | 6.05 | (6) |
| PE-Cys^{b} | 12.70 | (14) | 7.30 | (7) | 13.80 | (14) |
| Total | | (118) | | (59) | | (118) |

| | | | | | | |
|---|---|---|---|---|---|---|
| Values in brackets are those deduced from cDNA: rat ITF (Chinery et al., 1992) human ITF (Hauser et al., 1993). | | | | | | |
| ^{a}) Determined by extrapolation to zero time of hydrolysis | | | | | | |
| ^{b}) Determined by hydrolysis in 4M methane sulphonic acid. | | | | | | |
| ^{c}) Not determined | | | | | | |

**Table II Automated Edman Degradation of rat ITF (dimer), human ITF (monomer) and human ITF (dimer)**

| Cycle No. | Rat ITF (dimer) | | Human ITF (monomer) | | Human ITF (dimer) | |
|---|---|---|---|---|---|---|
| | PTA-AA. | Yield (pmol) | PTH-A.A. | Yield (pmol) | Yield (pmol) | |
| 1 | GIn | 989 | Glu | 1517 | Glu | 2227 |
| 2 | Glu | 731 | Glu | 1998 | Glu | 2361 |
| 3 | Phe | 967 | Tyr | 2205 | Tyr | 2528 |
| 4 | Val | 1072 | Val | 2409 | Val | 2431 |
| 5 | Gly | 701 | Gly | 1625 | Gly | 1803 |
| 6 | Leu | 838 | Leu | 2318 | Leu | 2253 |
| 7 | Set | 497 | Ser | 852 | Ser | 904 |
| 8 | Pro | 965 | Ala | 1729 | Ala | 1712 |
| 9 | Ser | 406 | Asn | 1394 | Asn | 1518 |
| 10 | Gln | 820 | Gln | 1494 | Gln | 1499 |
| 11 | (Cys) | n.d. | (Cys) | n.d. | (Cys) | n.d. |
| 12 | Met | 581 | Ala | 1243 | Ala | 1377 |
| 13 | Val | 971 | Val | 1468 | Val | 1356 |
| 40 | Pro | 962 | Pro | 1223 | Pro | 1195 |
| 15 | Ala | 529 | Ala | 1248 | Ala | 1249 |
| 16 | Asn | 791 | Lys | 1270 | Lys | 1050 |
| 17 | Val | 952 | Asp | 937 | Asp | 991 |
| 18 | Arg | 331 | Arg | 891 | Arg | 964 |
| 19 | Val | 956 | Val | 1002 | Val | 1069 |
| 20 | Asp | 476 | Asp | 847 | Asp | 932 |
| 21 | (Cys) | n.d. | (Cys) | n.d. | Cys | n.d. |
| 22 | Gly | 381 | Gly | 709 | Gly | 703 |
| 23 | Tyr | 352 | Tyr | 894 | Tyr | 792 |
| 24 | Pro | 927 | Pro | 766 | Pro | 701 |
| 25 | Thr | 498 | His | 309 | His | 236 |
| 26 | Val | 812 | Val | 755 | Val | 670 |
| 27 | Thr | 510 | Thr | 505 | Thr | 576 |
| 28 | Ser | 225 | PrQ | 458 | Pro | 473 |
| 29 | Glu | 398 | Lys | 444 | Lys | 321 |
| 30 | Gln | 499 | Glu | 219 | Glu | 304 |
| 31 | (Cys) | n.d. | (Cys) | n.d. | (Cys) | n.d. |
| 32 | Asn | 557 | Asn | 312 | Asn | 300 |
| 33 | Asn | 591 | Asn | 464 | Asn | 503 |
| 34 | Arg | 196 | Arg | 325 | Arg | 294 |
| 35 | Gly | 222 | Gly | 239 | Gly | 223 |
| 36 | (Cys) | n.d. | (Cys) | n.d. | (Cys) | n.d. |
| 37 | (Cys) | n.d. | (Cys) | n.d. | (Cys) | n.d. |
| 38 | Phe | 335 | Phe | 189 | Phe | 174 |
| 39 | Asp | 275 | Asp | 133 | Asp | 137 |
| 40 | Ser | 164 | Ser | 49 | Ser | 43 |
| R.Y. | | 97.0% | | 93.2% | | 92.5% |

### REFERENCES

Babyatsky, M.W., Thim, L., Podolsky, D.K. (1994) *Gastroenterology 106,* A43 (abstract).

Bruins, A.P., Covey, T.R., & Henion, J.D. (1987) *Anal. Chem.* 59, 2642-2646.

Chinery, R., Poulsom, R., Rogers, L.A., Jeffery, R.E. Longcroft, J.M., Hanby, A.M., & Wright, N.A. (1992) *Biocliem. J.* 285, 5-8.

Covey, T.R., Bonner, R.F., Shushan, B.L, & Henion, J.D. (1988) *Rapid Commun. Mass. Spectrom.* 2, 249-256.

Dignass, A., Lynch-Devaney, K., Kindon, H., Thim, L., & Podolsky, D.K. (1994) J. *Clin. Invest,* (in press).

Friedman, M., Krull, L.H., Cavins, J.F. (1970), *J. Biol. Chem.* 245, 3868-3871.

Gajhede, M., Petersen, T.N., Henriksen, A., Petersen, J.F.W., Dauter, Z., Wilson, K.S., & Thim, L. (1993) *Structure 1,* 253-262.

Hanby, A.M., Poulsom, R., Elia, G., Singh, S., Longcroft, J.M., & Wright, N.A. (1993) *J. Pathol.* 169, 355-360.

Hauser, F., & Hoffmann, W. (1991) *J. Biol. Chem.* 266,21206-21309.

Hauser, F., Roeben, C., & Hoffmann, W. (1992a) *J. Biol. Chem.* 267, 14451-14455.

Hauser, F., & Hoffmann, W. (1992b) *J. Biol. Chem.* 267, 24620-24624.

Hauser, F., Poulsom, R., Chinery, R., Rogers, L.A., Hanby, A.M., Wright, N.A., & Hoffmann, W. (1993) *Proc. Natl. Acad. Sci.* 90,6961-6965.

Hoffmann, W. (1988) *J. Biol. Chem. 263,* 7686-7690.

Hoffmann, W., & Hauser, F. (1993) *Trends Biochem. Sci.* 7, 239-243.

Jakowlew, S.B., Breathnach, R., Jeltsch, J.-M., Masiakowski, P., Chambon, P. (1984) *Nucleic Acid Res. 12,* 2861-2878.

Lefebvre, O., Wolf, C., Kedinger, M., Chenard, M.-P., Tomasetto, C., Chambon, P., & Rio, M.C. (1993) *J. Cell. Biol.* 122,191-198.

Playford, R.J., Marchbank, T., Chinery, R., Evison, R., Pignattelli, M., Bolton, R., Thim, L., & Hanby, A.M. (1994) *Gastroenterology* (in press).

Podolsky, D.K., Lynch-Devaney, K., Stow, J.L., Oates, P., Murgue, B., DeBeaumont, M., Sands, B.E., & Makida, Y.R. (1993) *J. Biol Chem. 268,* 6694-6702.

Poulsom, R., Chinery, R., Sarraf, C., Lalani, E.-N., Stamp, E., Elia, G., Wright, N. (1992) *Gastroenterology 27*, 17-28.

Poulsom, R., & Wright, N.A. (1993) *Am. J. Physiol. 265,* G205-G213.

Prud'homme, J.F., Fridlansky, F., Le Cunff, M., Atger, M., Mercier-Bodart, C., Pichon, M.-F., & Milgrom, E. (1985) *DNA 4,* 11-21.

Rio, M.C., Bellocq, J.P., Daniel, J.Y., Tomasetto, C., Lathe, R., Chenard, M.P., Batzenschlager, A., & Chambon, P. (1988) *Science 241,* 705-708.

Rio., M.-C., Chenard, M.-P., Wolf, C., Marcellin, L., Tomasetto, C., Lathe, R., Bellocq, J.-P., & Chambon, P. (1991) *Gastroenterology 100,* 375-379.

Rüegg, U. Th., & Rudinger, J. (1974) *Isr. J. Chem 12,* 391-401.

Sherman, F., Fink, G.R., & Hicks, J.B. (1981) Methods in yeast genetics, *Cold Spring Harbor Laboratory Press,* Cold Spring Harbor, NY.

Suemori, S., Lynch-Devaney, K., & Podolsky, D.K. (1991) *Proc. Natl. Acad. Sci. 88,* 11017-11021.

Thim, L., Jørgensen, K.H., & Jorgensen, K.D. (1982) *Regul. Peptides 3,* 221-230.

Thim, L., Thomsen, J., Christensen, M., & Jørgensen, K.H. (1985) *Biochem.,Biophys. Acta 827,* 410-418.

Thim, L., Hansen, M.T., Norris, K., Hoegh, I., Boel, E., Forstrom, J., Ammerer, G., & Fiil, L. (1986) *Proc. Natl. Acad. Sci. 83,* 6766-6770.

Thim, L., Hansen, M.T., & Soerensen, A.R. (1987) *FEBS Lett. 212,* 307-312.

Thim, L. (1989) *FEBS Lett. 250,* 85-90.

Thim, L., Norris, K., Norris, F., Nielsen, P.F., Bjørn, S.E., Christensen, M., Petersen, J. (1993) *FEBS Lett. 318,* 345-352.

Thim, L. (1994) *Digestion* 55, 353-360.

Tomasetto, C., Rio, M., Gautier, C., Wolf, C., Hareuveni, M., Chambon, P., & Lathe, R. (1990) *EMBO J. 9,* 407-414.

Wright, N.A., Pike, C., & Elia, G. (1990) *Nature 343,* 82-85.

Wright, N.A., Poulsom, R., Stamp, G., van Norden, S., Sarraf, C., Elia, G., Ahnen, D., Jeffery, R., Longcroft, J., Pike, C., Rio, M., & Chambon, P. (1993) *Gastroemerology 104,* 12-20.

## Claims

1. A trefoil peptide dimer **characterized by** being a dimer form of a trefoil peptide monomer, said monomer containing a single trefoil domain.

2. A trefoil peptide dimer according to claim 1 having an approximate molecular weight of 13000.

3. A trefoil peptide dimer according to claim 1 which is a dimer of intestinal trefoil factor (ITF).

4. A trefoil peptide dimer according to claim 1 or 3 which is a dimer of human ITF.

5. A trefoil peptide dimer according to claim 3 or 4, wherein the ITF dimer is composed of two ITF monomers linked by a disulphide bond between two cysteine residues in position 57 of each monomer.

6. A trefoil peptide dimer according to claim 1 which is a dimer of breast cancer associated peptide (pS2).

7. A trefoil peptide dimer according to claim 1 or 6 which is a dimer of human pS2.

8. A trefoil peptide dimer according to claim 6 or 7, wherein the pS2 dimer is composed of two pS2 monomers linked by a disulphide bond between two cysteine residues in position 58 of each monomer.

9. A method for preparing a trefoil peptide dimer **characterized by** being a dimer form of a trefoil peptide monomer, said monomer containing a single trefoil domain, the method comprising:
culturing a suitable host cell transformed with a DNA sequence encoding a trefoil peptide monomer containing one trefoil domain under conditions permitting production of the peptide;
separating the resulting trefoil peptide monomer and trefoil peptide dimer from the culture by a chromatographic procedure; and
recovering the trefoil peptide dimer.

10. A pharmaceutical composition comprising a trefoil peptide dimer **characterized by** being a dimer form of a trefoil peptide monomer, said monomer containing a single trefoil domain, together with a pharmaceutically acceptable diluent or vehicle.

11. A pharmaceutical composition according to claim 10, comprising a trefoil peptide dimer according to any one of claims 2-8.

12. A trefoil peptide dimer **characterized by** being a dimer form of a trefoil peptide monomer, said monomer containing a single trefoil domain, for use as a medicament.

13. A trefoil peptide dimer according to any one of claims 2-8 for use as a medicament.

14. A trefoil peptide dimer **characterized by** being a dimer form of a trefoil peptide monomer, said monomer containing a single trefoil domain, for use in the prophylaxis or treatment of gastrointestinal disorders.

15. A trefoil peptide dimer according to any one of claims 2-8 for use in the prophylaxis or treatment of gastrointestinal disorders.

16. A trefoil peptide dimer according to claim 14 or 15, wherein said gastrointestinal disorder is selected from:
gastric and peptic ulcers;
inflammatory bowel disease;
Crohn's disease; and
injury to the intestinal tract caused by radiation therapy, bacterial infection or other infections.

17. Use of a trefoil peptide dimer **characterized by** being a dimer form of a trefoil peptide monomer, said monomer containing a single trefoil domain, for the preparation of a medicament for the prophylaxis or treatment of gastrointestinal disorders.

18. The use according to claim 17, wherein said trefoil peptide dimer is a trefoil peptide dimer according to any one of claims 2-8.

19. The use according to claim 17 or 18, wherein said gastrointestinal disorder is selected from:
gastric and peptic ulcers;
inflammatory bowel disease;
Crohn's disease; and
injury to the intestinal tract caused by radiation therapy, bacterial infection or other infections.

## Patentansprüche

1. Dimer des Kleeblatt-Peptids, **dadurch gekennzeichnet, dass** es eine dimere Form eines Monomers des Kleeblatt-Peptids darstellt, wobei das Monomer eine einzelne Kleeblatt-Domäne enthält.

2. Dimer des Kleeblatt-Peptids nach Anspruch 1 mit einem Molekulargewicht von etwa 13000.

3. Dimer des Kleeblatt-Peptids nach Anspruch 1, das ein Dimer des Magen-Darm-Kleeblatt-Faktors (intestinal trefoil factor, ITF) ist.

4. Dimer des Kleeblatt-Peptids nach Anspruch 1 oder 3, das ein Dimer eines Human-ITF ist.

5. Dimer des Kleeblatt-Peptids nach Anspruch 3 oder 4, wobei das ITF-Dimer aus zwei ITF-Monomeren zusammengesetzt ist, die durch eine Disulfidbindung zwischen zwei Cysteinresten in Position 57 jedes Monomers miteinander verknüpft sind.

6. Dimer des Kleeblatt-Peptids nach Anspruch 1, das ein Dimer eines mit Brutkrebs verbundenen Peptids (pS2) ist).

7. Dimer des Kleeblatt-Peptids nach Anspruch 1 oder 6, das ein Dimer von Human-pS2 íst.

8. Dimer des Kleeblatt-Peptids nach Anspruch 6 oder 7, wobei das pS2-Dimer aus zwei pS2-Monomeren zusammengesetzt ist, die durch eine Disulfidbindung zwischen zwei Cysteinresten in Position 58 jedes Monomers miteinander verknüpft sind.

9. Verfahren zur Herstellung eines Dimers des Kleeblatt-Peptids, **dadurch gekennzeichnet, dass** es eine dimere Form eines Monomers des Kleeblatt-Peptids ist, wobei das Monomer eine einzelne Kleeblatt-Domäne enthält, wobei das Verfahren Folgendes umfasst:
Züchten einer geeigneten Wirtszelle, die mit einer DNA-Sequenz transformiert ist, die ein eine Kleeblatt-Domäne enthaltendes Monomer des Kleeblatt-Peptids kodiert, unter die Herstellung des Peptids gewährenden Bedingungen;
Abtrennen des erhaltenen Monomers des Kleeblatt-Peptids und Dimer des Kleeblatt-Peptids von der Kultur durch ein chromatografisches Verfahren; und
Gewinnen des Dimers des Kleeblatt-Peptids.

10. Arzneimittel, umfassend ein Dimer des Kleeblatt-Peptids, **dadurch gekennzeichnet, dass** es eine dimere Form eines Monomers des Kleeblatt-Peptids ist, wobei das Monomer eine einzelne Kleeblatt-Domäne enthält, zusammen mit einem pharmazeutisch verträglichen Verdünnungsmittel oder Vehikulum.

11. Arzneimittel nach Anspruch 10, umfassend ein Dimer des Kleeblatt-Peptids nach einem der Ansprüche 2-8.

12. Dimer des Kleeblatt-Peptids, **dadurch gekennzeichnet, dass** es eine dimere Form eines Monomers des Kleeblatt-Peptids ist, wobei das Monomer eine einzelne Kleeblatt-Domäne enthält, zur Verwendung als Medikament.

13. Dimer des Kleeblatt-Peptids nach einem der Ansprüche 2-8 zur Verwendung als Medikament.

14. Dimer des Kleeblatt-Peptids, **dadurch gekennzeichnet, dass** es eine dimere Form eines Monomers des Kleeblatt-Peptids ist, wobei das Monomer eine einzelne Kleeblatt-Domäne enthält, zur Verwendung bei der Prophylaxe oder Behandlung von Magen-Darm-Störungen.

15. Dimer des Kleeblatt-Peptids nach einem der Ansprüche 2-8 zur Verwendung bei der Prophylaxe oder Behandlung von Magen-Darm-Störungen.

16. Dimer des Kleeblatt-Peptids nach Anspruch 14 oder 15, wobei die Magen-Darm-Störung ausgewählt ist aus:
Magen- und Zwölffingerdarmgeschwüren;
entzündlicher Darmerkrankung;
Crohn-Krankheit; und
Verletzung des Darmtrakts, verursacht durch Strahlungstherapie, Bakterieninfektion oder anderen Infektionen.

17. Verwendung eines Dimers des Kleeblatt-Peptids, **dadurch gekennzeichnet, dass** es eine dimere Form eines Monomers des Kleeblatt-Peptids ist, wobei das Monomer eine einzelne Kleeblatt-Domäne enthält, zur Herstellung eines Medikaments zur Prophylaxe oder Behandlung von Magen-Darm-Störungen.

18. Verwendung nach Anspruch 17, wobei das Dimer des Kleeblatt-Peptids ein Dimer des Kleeblatt-Peptids nach einem der Ansprüche 2-8 ist.

19. Verwendung nach Anspruch 17 oder 18, wobei die Magen-Darm-Störung ausgewählt ist aus:
Magen- und Zwölffingerdarmgeschwüren;
entzündlicher Darmerkrankung;
Crohn-Krankheit; und
Verletzung des Darmtrakts, verursacht durch Strahlungstherapie, Bakterieninfektion oder anderen Infektionen.

## Revendications

1. Dimère peptidique en trèfle **caractérisé en ce qu'**il est sous une forme dimère d'un monomère peptidique en trèfle, ledit monomère contenant un domaine en trèfle unique.

2. Dimère peptidique en trèfle selon la revendication 1, ayant un poids moléculaire approximatif de 13 000.

3. Dimère peptidique en trèfle selon la revendication 1, qui est un dimère du facteur intestinal en trèfle (ITF).

4. Dimère peptidique en trèfle selon la revendication 1 ou 3, qui est un dimère de ITF humain.

5. Dimère peptidique en trèfle selon la revendication 3 ou 4, dans lequel le dimère ITF est constitué de deux monomères ITF liés par un pont disulfure entre deux résidus cystéine à la position 57 de chaque monomère.

6. Dimère peptidique en trèfle selon la revendication 1, qui est un dimère d'un peptide associé au cancer du sein (pS2).

7. Dimère peptidique en trèfle selon la revendication 1 ou 6, qui est un dimère de pS2 humain.

8. Dimère peptidique en trèfle selon la revendication 6 ou 7, dans lequel le dimère pS2 est constitué de deux monomères pS2 liés par un pont disulfure entre deux résidus cystéine à la position 58 de chaque monomère.

9. Procédé pour préparer un dimère peptidique en trèfle **caractérisé en ce qu'**il est sous une forme dimère d'un monomère peptidique en trèfle, ledit monomère contenant un domaine en trèfle unique, le procédé comportant les étapes consistant à :
cultiver une cellule hôte appropriée transformée par une séquence d'ADN codant pour un monomère peptidique en trèfle contenant un domaine en trèfle sous des conditions permettant la production du peptide,
séparer le monomère peptidique en trèfle résultant et le dimère peptidique en trèfle de la culture par un procédé chromatographique, et
récupérer le dimère peptidique en trèfle.

10. Composition pharmaceutique comportant un dimère peptidique en trèfle, **caractérisée en ce qu'**il est sous une forme dimère d'un monomère peptidique en trèfle, ledit monomère contenant un domaine en trèfle unique, ensemble avec un diluant ou véhicule pharmaceutiquement acceptable.

11. Composition pharmaceutique selon la revendication 10, comportant un dimère peptidique en trèfle selon l'une quelconque des revendications 2 à 8.

12. Dimère peptidique en trèfle **caractérisé en ce qu'**il est sous une forme dimère d'un monomère peptidique en trèfle, ledit monomère contenant un domaine en trèfle unique, destiné à être utilisé en tant que médicament.

13. Dimère peptidique en trèfle selon l'une quelconque des revendications 2 à 8, destiné à être utilisé en tant que médicament.

14. Dimère peptidique en trèfle **caractérisé en ce qu'**il est sous une forme dimère d'un monomère peptidique en trèfle, ledit monomère contenant un domaine en trèfle unique, destiné à être utilisé pour la prophylaxie ou le traitement de troubles gastro-intestinaux.

15. Dimère peptidique en trèfle selon l'une quelconque des revendications 2 à 8, destiné à être utilisé pour la prophylaxie ou le traitement de troubles gastro-intestinaux.

16. Dimère peptidique en trèfle selon la revendication 14 ou 15, dans lequel ledit trouble gastro-intestinal est sélectionné parmi :
des ulcères gastrique et gastro-duodénal,
une maladie inflammatoire de l'intestin,
la maladie de Crohn, et
une lésion de la voie intestinale provoquée par une thérapie par rayonnement, une infection bactérienne ou autres infections.

17. Utilisation d'un dimère peptidique en trèfle **caractérisée en ce qu'**il est sous une forme dimère d'un monomère peptidique en trèfle, ledit monomère contenant un domaine en trèfle unique, pour la préparation d'un médicament destiné à la prophylaxie ou au traitement de troubles gastro-intestinaux.

18. Utilisation selon la revendication 17, dans laquelle ledit dimère peptidique en trèfle est un dimère peptidique en trèfle selon l'une quelconque des revendications 2 à 8.

19. Utilisation selon la revendication 17 ou 18, dans laquelle ledit trouble gastro-intestinal est sélectionné parmi :
des ulcères gastrique et gastro-duodénal,
une maladie inflammatoire de l'intestin,
la maladie de Crohn, et
une lésion de la voie intestinale provoquée par une thérapie par rayonnement, une infection bactérienne ou autres infections.
